# EUROPEAN PATENT SPECIFICATION

(11) **EP 1 722 717 B1**
(45) Date of publication and mention of the grant of the patent: **19.06.2013**
(21) Application number: 04794020.0
(22) Date of filing: 04.10.2004
(51) Int. Cl.: A61L 27/52, A61F 2/30

(54) **HYDROGELS HAVING CHARGED SURFACES FOR CARTILAGE REPLACEMENT**
HYDROGELE MIT GELADENEN OBERFLÄCHEN FÜR DEN KNORPELERSATZ
HYDROGELS A SURFACES CHARGEES DESTINES A REMPLACER UN CARTILAGE

(30) Priority: 02.10.2003 US 677444; 14.04.2004 US 562176 P
(43) Date of publication of application: 22.11.2006
(73) Proprietor: Mansmann, Kevin A., Paoli, PA 19301 (US)
(72) Inventor: Mansmann, Kevin A., Paoli, PA 19301 (US)
(74) Representative: Ascherl, Andreas
(86) International application number: PCT/US2004/032504
(87) International publication number: WO 2005/032426

(56) References cited:
- EP-A1- 0 677 297
- US-A- 4 183 884
- US-A- 4 836 884
- US-A- 5 314 478
- US-A- 5 795 353
- US-A1- 2002 022 884
- US-A1- 2003 036 801
- US-A1- 2003 212 456
- US-A1- 2004 070 107
- US-A1- 2004 070 107

## Description

### BACKGROUND OF THE INVENTION

This invention relates to surgical implants for replacing or repairing hyaline cartilage, in joints such as knees, hips, shoulders, etc. As used herein, all references to implants, surgery, etc., refer to surgical (which includes arthroscopic) implantation of a device into a mammalian joint.

As known in the art, hydrogels are materials that are somewhat flexible and pliable, and do not have rigid or crystalline structures. In hydrated form, they contain water molecules, which can permeate through a matrix (i.e., three-dimensional network) of flexible crosslinked fibers. In animals, nearly all types of soft tissues are hydrogels, with matrices made of collagen (a bundled protein that provides tensile strength) and proteoglycan filaments (extremely thin protein strands surrounded by hyaluronate, a natural polymer).

Because natural tissues are hydrogels, many efforts have been made to use hydrogels as cell culture materials. While these materials have numerous laboratory uses, the use of hydrogel implants to replace injured or diseased cartilage, in surgery on humans, has been very limited, for a number of reasons. The only sales of such hydrogel implants that are known to the Applicant are occurring in Europe, by a business venture involving Salumedica (a European company) and Arthrex (an American company). Those implants are believed to be limited to relatively small "plugs" being used and tested to see whether they can repair relatively small cartilage defects. That approach suffers from shortcomings that limit its utility and effectiveness, notably including problems involving minor edges and noncomformities that lead to potentially abrasive surfaces around the periphery of any such inserted plug surrounded by cartilage. Also, those implants are believed to be made of polyvinyl alcohol (PVA), which is not as strong or durable as other known hydrogel materials.

Another important distinction should be noted between those efforts, and the approach described herein. In essentially all cases, the implants described herein will be designed to completely replace an entire segment of cartilage (such as an entire femoral runner, tibial plateau, or patellar surface), rather than attempting to insert a small plug or disc of synthetic hydrogel into a defect that will remain surrounded by natural cartilage.

Most hydrogels described in science or medical articles include hydrogels made of collagen, the natural protein that provides the matrix that surrounds and supports cells in nearly all soft tissues in animals. However, collagen hydrogels suffer from problems and limitations, if used in implants for replacing cartilage. Those problems include: (1) the risk that a foreign protein will provoke a tissue rejection, especially if the protein is from a non-human source such as cowhide (the source of most collagen available for testing and use); (2) collagen fibers are typically digested, resorbed, and replaced within a span of months, as part of natural tissue regeneration processes; (3) toxic chemicals are usually needed to crosslink collagen fibers in ways that will create matrices; and, (4) collagen has less strength and durability than various types of known synthetic polymers.

Accordingly, the Applicant has avoided collagen, and has focused instead on synthetic polymers for creating implants designed to last at least 10 years (and preferably for the entire remaining life of the patient, especially in the case of elderly patients).

Although certain synthetic hydrogels (such as polyhydroxy-ethyl-methacrylate) are used for contact lenses and slow-release drug carriers, they are not strong or durable enough to replace hyaline cartilage. Other biocompatible polymers are described in patents such as US 3,822,238 (Blair et al 1974), 4,107,121 (Stoy 1978), 4,192,827 (Mueller et al 1980), 4,424,305 (Gould et al 1984), 4,427,805 (Stol et al 1984), and 4,563,490 (Stol et al 1986), and methods of coating hydrogel layers onto harder "substrate" materials are described in patents such as US 4,921,497 (Sulc et al 1990) and 5,688,855 (Stoy et al 1997).

However, none of those are being used to replace hyaline cartilage in load-bearing joints, such as knees or hips. Instead, knee and hip replacements today use rigid metallic and plastic components. In a typical knee implant, the tibial bone (in the shin) is sawed off below the knee, and the entire upper segment of bone is replaced with a titanium alloy piece, with a high-density polyethylene (HDPE) plastic coating on the upper condyle, to provide the tibial plateau. The lower end of the femur (in the thigh) is also sawed off, and replaced by a hard metal piece having rounded "runners" made of a cobalt chrome alloy. Similarly, in a hip replacement, the surface of the acetabular socket of the pelvic bone is cut and grinded away, and replaced by a titanium piece with a polyethylene coating. The upper head of the femur is also sawed off, and replaced with a metal piece having a rounded surface made of cobalt chrome.

These operations inflict severe damage on the muscles, tendons, ligaments, bones, and blood vessels, in and around a knee or hip. They cause severe pain, and since they inflict so much damage on tissues and vasculature, many elderly people never fully recover from these surgeries. Less invasive surgery, using smaller flexible implants that could be inserted through smaller incisions, would provide major advantages, if such implants could be strong enough.

The problems that render hydrogels too weak and fragile for use in knee or hip replacements have arisen because, in a typical hydrogel, the fibers that hold the gel together take up only a small portion of the volume (usually less than about 10%, and many hydrogels contain less than 5% fiber volume). Even in relatively dense and tough synthetic hydrogels, the polymeric strands usually take up only about 30% of the volume, while the remaining 70% is "interstitial" space (i.e., gaps within the fibrous matrix). When a gel is hydrated, those interstitial spaces are filled with water molecules. Since water molecules cannot contribute any significant strength to a hydrogel, large loads must be imposed on the fibers that form the matrix and hold the water molecules together.

The problems of low strength and durability are also aggravated by the lack of a crystalline structure in a hydrogel. If a hydrogel material had a crystalline structure, with repeating units in regular rows and columns that could reinforce the matrix, it would be stronger, but it would not be adequately flexible and resilient. Instead, hydrogels use long organic molecular chains that are not straight, and instead have "zig-zag" structures, usually with angles of about 110 degrees between adjacent bonds on each carbon atom. These "zigzagging" polymer chains are useful, since they allow the molecules to be either compressed or stretched in an elastic and springy manner without breaking, but they cannot provide the type of reinforcement or strength that could be provided by crystalline lattices.

Crosslinking attachments that connect hydrogel molecules to each other also contribute to the elasticity and pliability of the gels. These crosslinking attachments do not occur at close intervals, or high densities. Instead, they occur semi-randomly, in ways usually described by average distances between adjacent crosslinking bonds. A typical hydrogel polymer will usually have crosslinking groups spaced apart from each other by about 3 to 10 atoms, and to provide even more permeability for water molecules, the crosslinking groups often are attached to side-chains that are several atoms long.

Because the low strength and durability of hydrogels is well known, and limits their utility, there have been numerous efforts to create stronger hydrogels. These efforts generally fall into two categories. One set of efforts involves the chemistry of the polymeric strands that form a hydrogel. At this time, two chemical approaches for making relatively strong, tough, and durable hydrogels involve: (1) polyacrylonitrile mixtures, as described in patents such as US 4,420,589 (Stoy 1983), 4,493,618 (Stoy et al 1990), 5,688,855 (Stoy et al 1997), and 6,593,451 (Stoy 2003); and (2) hydrophilic polyurethanes, described in items such as US patent 4,424,305 (Gould et al 1984) and Gorman et al 1998.

Polyacrylonitrile compounds have been the subject of most of the research efforts to date by the Applicant herein, because they offer promising levels of toughness and durability, even when formulated as permeable and lubricious hydrogels.

However, polyurethane compounds also merit close and careful attention, because they can be formulated in various ways that will create hydrogels. As a brief introduction, most polyurethane compounds are created by mixing a resin with a catalyst. The resin has the general formula HO-X-OH, where X is a variable that represents any organic molecule; since a hydroxy group (-OH) coupled to a carbon atom creates an alcohol, this resin can be referred to as an alcohol resin. The catalyst has at least one cyanate group (O=C=N-). To enable polymerization, most catalysts have at least two cyanate groups, which flank an organic atom or group, represented by the variable Y in the formula O=C=N-Y-N=C=O.

When cyanate groups in a catalyst react with alcohol groups in a resin, the result is a polyurethane compound, which has a repetitive linkage and bonding structure as follows 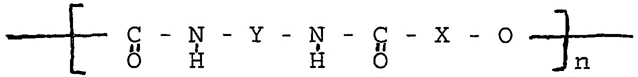 where "n" represents the average number of "monomer" units that were linked together to form the polymerized molecules.

Three aspects of these resin and catalyst reagents should be noted:
(1) The "X" variable, contributed by the alcohol resin, can have any desired structure, such as a branching structure with multiple hydroxy or other hydrophilic groups at the tips of some or all of the branches. If a branched resin with multiple hydroxy groups is used (such resins are often called "polyol" resins), it can create molecular matrices with more complex structures than can be achieved by linear resins. In addition, a resin with branched constituents can have various different types of reactive groups at the tips of some of the branches, to allow still more controllable options.
(2) The Y variable, contributed by the cyanate catalyst, also can provide side chains and/or branched groups, allowing still more ways to modify and control the structure, content, and performance of resulting polyurethane materials.
(3) Blends of different resins and/or different catalysts can be mixed together, to provide even more options and controllable performance traits.

By utilizing these and other options, researchers and companies have developed polyurethane compounds into a broad class of highly adaptable and useful polymers that can be given a wide variety of structures and performance traits. Therefore, polyurethanes merit careful attention as candidate hydrogels for testing and evaluation as described herein.

The other class of efforts to create stronger and more durable hydrogels involves the use of reinforcing fibers, embedded within a hydrogel. Such efforts are described in articles such as Corkhill et al 1989, Blue et al 1991, Walker et al 1991, and Ambrosio et al 1998. However, none of those efforts have led to any successful hydrogel implants for load-bearing joints such as knees or hips.

One class of fiber-reinforced hydrogels deserves attention in passing, but it is not relevant to any efforts to replace cartilage in articulating joints. Researchers have tried to develop fiber-reinforced hydrogels for repairing or replacing damaged spinal discs. Unlike hyaline cartilage in articulating joints, which have smooth and slippery surfaces, spinal discs are made of a completely different type of "fibro-cartilage". They do not have smooth and slippery "articulating" surfaces, because they must not allow any sliding motion to occur, between vertebral bones. Indeed, one of their most crucial functions is to prevent and prohibit any sliding motion between vertebrae, because any transverse sliding motion, in a spine, would injure and possibly even shear (i.e., transversely cut) the spinal cord. Therefore, spinal discs evolved with a type of cartilage that has long fibers extending outwardly from the planar surfaces of each disc. Those fibers extend well into the vertebral bones, to form strong transition zones that prevent any sliding motion that might otherwise pinch or shear the spinal cord. By contrast, hyaline cartilage has a totally different structure that actively promotes smooth, slippery, lubricated sliding motion.

Therefore, efforts to develop non-sliding hydrogels for spinal repair (such as US patents 4,911,718, Lee et al 1990, and 5,171,281, Parsons et al 1992) are not relevant to efforts to replace sliding cartilage in articulating joints. Accordingly, any references herein to cartilage, implants, or similar terms are limited to devices having at least one smooth articulating surface (this includes hyaline or "condylar" cartilage, as well as meniscal or labral cartilage), and excludes implants designed for spinal repair.

Smooth-surfaced implants made of flexible plastics (typically silicone rubber, rather than hydrogels) are sometimes used to replace cartilage in joints that do not endure high loadings, such as in wrists or fingers, and hydrogel implants as disclosed herein can be adapted for use in such joints, if desired. However, the loadings, stresses, and wear that are imposed on fingers, wrists, or similar joints (even including surgically-repaired shoulders) do not begin to approach the levels of loading, stress, and wear imposed on cartilage in knees and hips. Therefore, any prior art that is limited to hydrogel implants developed for low-stress joints (such as wrists or fingers) is not deemed relevant to cartilage replacement implants in joints such as knees or hips, which provide very different challenges and design constraints.

One other distinction should also be noted. Any use herein of terms such as matrix, mesh, fiber, fibrous, or strand, is intended to refer to a matrix made of fibers that will readily flex and bend, at room or body temperatures. This is pointed out, because the term "fiber mesh" is sometimes used to describe entirely different types of implants. Many implants designed to be anchored to bones have porous anchoring surfaces, to promote ingrowth of tissue into the implants (which promotes stronger anchoring of the implant to a bone). These porous surfaces are often made of thin strands of titanium or other hard metal, compressed into final shape while the metal was hot enough to be soft and nearly molten. Since patterns formed by the thin metal strands can be seen in the surfaces of these implants, they are often referred to as having "fiber mesh" surfaces (e.g., US patent 5,314,478, Oka et al 1994). However, rigid implants made of very hard metals, for promoting bony tissue ingrowth and anchoring, are entirely different from hydrogel matrices made of flexible thread-like strands. Accordingly, terms herein such as fiber, fibrous, matrix, mesh, etc., refer to fibers that are readily flexible, and easily bent, at body temperatures. These types of matrices, and methods of manufacturing them, are discussed below.

### Applicant's Closest Known Art

The closest known art in this field of research is the Applicant's own prior published patent application, published in November 2002 as US 2002/173855 (arising from serial number 10/071,930). That application disclosed an earlier version of an implant that contained some but not all of the components and features disclosed herein. Briefly, that application disclosed a flexible implant having the following components:
(1) an articulating surface made of a smooth hydrogel;
(2) an internal reinforcing mesh, which approached but did not cover or roughen the hydrogel surface;
(3) a multi-perforated non-planar interface layer (also called a "perforated waffle" layer), which provided a reinforcing interface between the soft gel material and the harder anchoring layer; and,
(4) a porous anchoring layer, which is placed against a prepared hard bone surface from which the cartilage has been removed, and which promotes ingrowth of bony tissue into the anchoring layer.

That assemblage still needed work on various aspects (such as, for example, detailed design of an anchoring system with enough strength and security to reliably prevent it from failing, even 20 years or more after implantation, without allowing any of the anchoring devices to disrupt the smoothness of a hydrogel surface that would be just a few millimeters away). Nevertheless, it was regarded as a substantial advance in the art, so it was disclosed to several companies that manufacture and sell cartilage-replacing implants, in the hope that at least one of those companies would recognize its potential, and have its specialists and consultants develop it into fully functional prototypes that would be fabricated and tested in animals.

Although some of those companies expressed interest, all of them declined to pursue it. Therefore, the Applicant continued to do additional research on his own, with limited grant funding from regional not-for-profit organizations.

In the course of that work, he created additional enhancements that have substantially improved the utility, strength, durability, and practicality of the complete device. Therefore, this application discloses a more refined, advanced, and complete system, including a number of enhancements that were not described in published US application 2002/173855.

Additional relevant art and background information can also be found in US patents 6,530,956 (Mansmann 2003), which relates to a resorbable fibrous matrix that can be used to protect transplanted cartilage-generating cells after they have been implanted into a joint, and 6,629,997 (also Mansmann 2003), which relates to wedge-shaped reinforced hydrogels that can be used as meniscal or labral implants. The '997 patent discusses meniscal implants with reinforcing mesh, but it did not disclose anything about chemical surface treatments, negative charge densities, or anchoring means as disclosed herein. The '956 resorbable matrix patent contains a Figure 6 that is worth noting, and accompanying text that begins under the heading, "Selectively Permeable Outer Membranes" in column 18. That passage describes lubricin and surface-active phospholipids (SAPL), as well as other major components of synovial fluid, but it does not say anything about the electrical charges or charge densities on lubricin or cartilage surfaces, which have recently been recognized to be important factors, as discussed below in the "Detailed Description" section.

Other recent prior art can be found in US patents 6,626,945 and 6,632,246 (both by Simon et al 2003, assigned to ChondroSite LLC). The '246 patent describes various types of laminated anchoring plugs, having three distinct layers that are bonded to each other, as described in column 16, lines 1-35. Although these implants do not attempt to create hydrogels at all, they nevertheless depict a current and ongoing effort in this field, by an entire research team, involving polyurethane compounds, which were mentioned above (along with PAN materials) as offering promising candidate synthetic polymers.

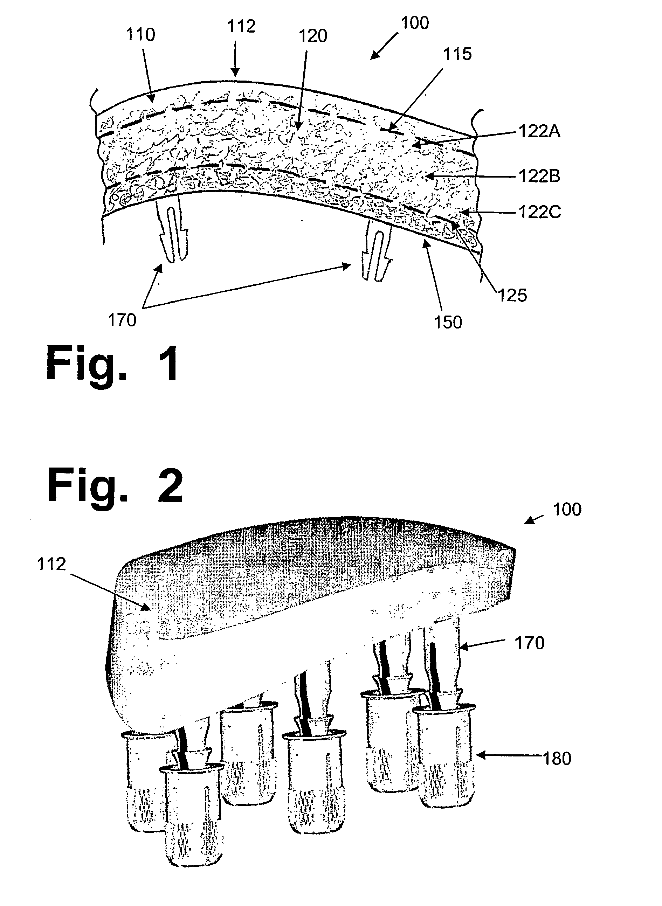

The lower (distal) end on one of the anchoring plugs of Simon et al, which will be inserted into the bone and which is depicted by callout number 54 in FIG. 1, is made of a relatively hard bone-like material, such as a polycarbonate polyurethane blend referred to as "75-D", which has "an elastic modulus similar to subchondral bone". A middle layer, shown by callout number 56 in FIG. 1, is made of a polycarbonate polyurethane blend referred to as "55-D", and has "properties similar to natural cartilage". The upper (proximal) surface, which will provide the exposed articulating surface after implantation of the plug, is made of "polycarbonate polyurethane 80-A or a thermoplastic hydrogel coating, which has properties similar to those of hyaline cartilage." As stated in Column 16, the different layers of these laminated plugs can be held together by "polyurethane adhesives that contain non-leachable isocyanate groups".

That proposed type of lamination, which involves gluing together layers of different materials, does not provide for (or even allow for) reinforcing meshes to be included and incorporated into the layers, in ways that will span the boundaries between the layers. A complete reading of both patents, supplemented by computerized searches for terms such as fiber, fibrous, mesh, or matrix in the searchable text versions of both patents that were posted on the U.S. Patent Office website, did not reveal any mention or suggestion of any type of reinforcing fibrous mesh, to strengthen either the individual layers themselves, or the interfaces and boundaries between those layers. In addition, all of the drawings that disclose such layers clearly indicate that the layers will be glued together using what effectively will be flat and planar interfaces.

Flat and planar gluing or adhesion surfaces may be adequate, for certain types of very strong polymers (including polyurethane) that will not fill up and swell with water to become hydrogels. However, flat and planar gluing or adhesion surfaces are not adequate for bonding a hydrogel material to a hard anchoring material, in a surgical implant that will need to have a reliable design life of at least 10 or preferably 20 years (or even longer), in a knee or other load-bearing joint. Even the best known hydrogel materials simply are not strong enough, and durable enough, to provide numerous years or even decades of reliable service in a load-bearing joint such as a human knee, if a pre-formed piece of hydrogel is merely glued to the top of a surface of harder material. Accordingly, mesh-reinforced composite hydrogel implants as disclosed herein are substantially different from, and are not suggested by, a glued-together laminated implant as taught by US patents 6,626,945 and 6,632,246.

Accordingly, one object of this invention is to disclose an improved composite hydrogel implant device for surgical replacement of damaged or diseased hyaline cartilage, containing at least one smooth and lubricious articulating surface, and having an internal flexible reinforcing mesh, and having a porous yet flexible anchoring surface, which provides an integrated unit having greater strength and durability than any previously known flexible implant having a hydrogel articulating surface.

Another object of this invention is to disclose a composite implant having a hydrogel articulating surface that has been chemically treated in a way that renders it more lubricious and durable, and that imparts to the hydrogel surface a negative charge density that emulates the negative charge on natural cartilage.

Another object of this invention is to disclose a surgical implant having a hydrogel component that partially encloses a flexible fibrous matrix, wherein the articulating surface of the hydrogel is covered by a layer treated by sulfonation or other chemical means, to provide a more lubricious and durable articulating surface having a negative charge.

Another object of this invention is to disclose a composite reinforced hydrogel implant with an improved anchoring system, comprising pegs that can be securely affixed in accommodating receptacles that have been securely inserted into prepared recesses, in hard bone, prior to insertion of the composite hydrogel implant.

Another object of this invention is to disclose a composite reinforced hydrogel implant that has sufficient strength and durability for use in replacing hyaline cartilage in a load-bearing joint, without requiring an internal reinforcing layer that poses a danger of damaging the hydrogel portion of the implant if the recipient suffers a fall or accident that causes a very high instantaneous loading.

These and other objects of the invention will become more apparent through the following summary, drawings, and description of the preferred embodiments.

### SUMMARY OF THE INVENTION

According to one aspect of the present invention the underlying problem is solved by providing a surgical implant according to claim 1 According to another aspect of the present invention, the underlying problem is solved by providing a method according to claim 12. Advantageous embodiments can be implemented according to the dependent claims.

Thus, hydrogel device for surgical implantation to replace damaged hyaline or meniscal cartilage in a mammalian joint is disclosed, with a combination of enhancements and improvements over previous proposed implants. One improvement comprises a hydrogel surface that has been chemically treated, by sulfonation or other means, to give it a negative electrical charge that emulates natural cartilage and improves its interactions with certain components of synovial fluid. Another improvement comprises a porous anchoring surface provided with anchoring pegs that will lock in place when pressed into receptacles that have been set and anchored in hard bone, prior to insertion of the implant. A third improvement comprises eliminating a non-planar plastic interface layer that posed a risk of lacerating or puncturing the hydrogel layer, and replacing that plastic layer with transitional gradients between the fibrous mesh and the porous anchoring layer. These improvements, when combined into a single unitary device, can increase the strength and durability of composite hydrogel implants to a level that will enable their use as relatively small and thin yet permanent implants in joints that require cartilage replacement, including load-bearing joints such as knees or hips. These implant devices can be flexible, to allow insertion through arthroscopic tubes and minimally-invasive incisions.

### BRIEF DESCRIPTION OF THE DRAWINGS

FIGURE 1 is a cross-sectional depiction of a cartilage-replacing implant as disclosed herein, showing a hydrogel having a smooth and lubricious chemically-treated surface, a flexible reinforcing mesh beneath the articulating surface, and a porous anchoring layer that promotes tissue ingrowth, and having anchoring pegs that will lock in place when pressed into receptacles that have been set into hard bone before insertion of the implant.
FIGURE 2 is a perspective view of a condylar implant as shown in FIG. 1, also showing the receptacles that will be anchored in hard bone.
FIGURE 3 depicts two implants that will articulate against each other, showing a uni-compartmental femoral runner implant positioned above a tibial plateau implant having a meniscal wedge portion around its outer periphery.
FIGURE 4 depicts the underside of a tibial plateau implant, showing a plurality of anchoring pegs in a spaced arrangement.
FIGURE 5 is a cross-sectional cutaway view of a surface-treated wedge-shaped implant for replacing a meniscal or labral segment of cartilage.

### DETAILED DESCRIPTION

As summarized above, hydrogel implants for replacing damaged or diseased cartilage in mammalian joints are disclosed, having enhancements that render them stronger, more lubricious, and more durable than any previously known hydrogel implants. These implants are believed to have enough strength and durability for use in load-bearing joints such as knees and hips, and they also can be adapted (such as by proper sizing) for use in other joints such as shoulders, wrists, fingers, etc.

The layers and zones that comprise implant 100 are shown in a cutaway view in FIG. 1. Implant 100 comprises a hydrophilic polymer that forms a flexible hydrogel when saturated with an aqueous solution, such as physiological saline. Several synthetic polymers are known that have levels of toughness and durability that make them preferred candidates for evaluation, include certain types of polyacrylate compounds and derivatives (including polyacrylonitrile (PAN) compounds, regarded as one category of polyacrylate compounds), and polyurethane hydrogels. As described below, such candidate materials preferably should be manufactured and/or surface-treated in ways that will give them negative charge densities, on their articulating surfaces, which will emulate the charge density of natural cartilage and promote beneficial interactions with positively-charged components of mammalian synovial fluid.

Implant 100 has three main layers, which preferably should have gradient-type transition zones between them, rather than abrupt planar boundaries. Surface layer 110 has a completely smooth and lubricious articulating surface 112, which has been chemically treated (such as by sulfonation) in a way that extends down to a depth or transition zone represented by dotted line 115. Interior layer 120 (and a portion of surface layer 110) is reinforced with a flexible fibrous mesh 122, which can have gradient zones if desired, such as a relatively low density zone 122A (to allow maximum permeability for water molecules), a medium density zone 122B, and a heavy density zone 122C which forms an interface 125 with porous anchoring layer 150. Anchoring layer 150 will directly contact a prepared bone surface after implantation, and is made of a porous material that promotes tissue ingrowth, for stronger anchoring.

Anchoring pegs 170 are securely affixed to anchoring layer 150. As illustrated in FIG. 2, anchoring pegs 170 will lock into place when pressed into accommodating receptacles 180, which are designed to be inserted and securely affixed (such as by cementing, threadings, etc.) into recesses that will be drilled or otherwise prepared in hard bone that will support the implant. Insertion and placement of the anchoring receptacles 180 preferably should be carried out prior to insertion of the implant 100 into the joint, as described below.

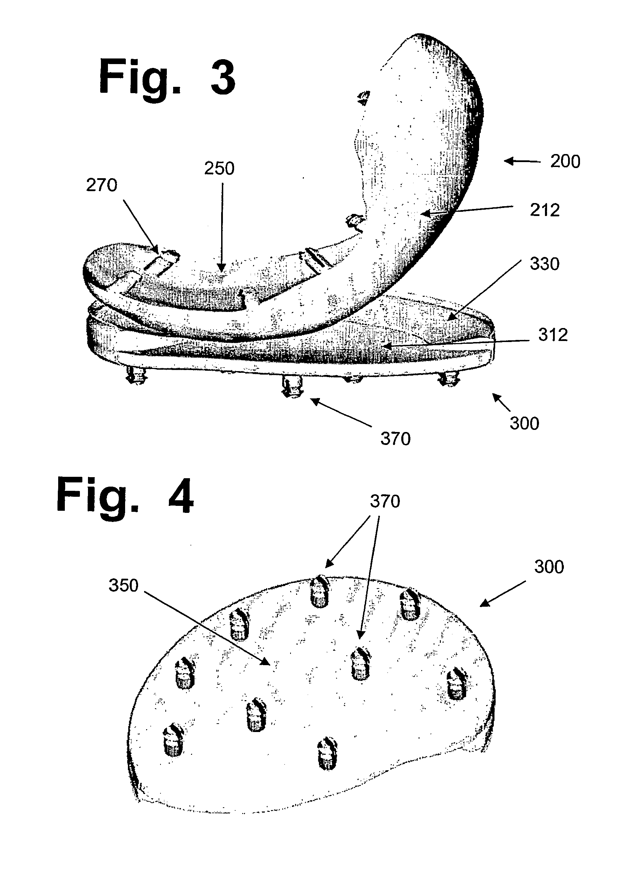

FIG. 3 illustrates a uni-compartmental implant for a knee joint, comprising femoral runner implant 200, and tibial plateau implant 300. Femoral implant 200 has a smooth articulating surface 212, anchoring layer 250, and anchoring pegs 270. Tibial implant 300 has a smooth articulating surface 312, a meniscal replacement wedge 330 molded into the implant's outer (lateral) periphery, and anchoring pegs 370, with a distribution such as shown in FIG. 4.

Because an earlier embodiment of this same general type of implant (invented by the same Applicant herein) has been published on the U.S. Patent Office website, as patent application 2002/173855 (arising from serial number 10/071,930), the discussion below will regard the teachings of that application as a starting or "baseline" point, and will focus on modifications and improvements that were created after that application was filed.

### CHEMICAL TREATMENT OF HYDROGEL SURFACES

During his research into candidate polymers that might be used to make hydrogel implants, the Applicant encountered a number of articles and patents discussing polyacrylonitrile (PAN). Various patents in that field were obtained in the 1970's and 1980's by Otto Wichterle and Artur Stoy, in Prague. While reviewing the titles and abstracts of all patents issued to those inventors, the Applicant noticed a mention of sulfuric acid treatment of hydrogel tubes, in US patent 4,183,884 (1980).

That patent relates to cannulas and catheters, which are tubes used to drain seepage and fluids from an injured or surgically repaired portion of a patient's body, or to drain urine from the bladder for a patient who will be bedridden and unable to get to a toilet. When made of hydrophobic plastics such as polyvinyl chloride, those types of tubes commonly cause irritation, inflammation, or other problems. Therefore, Wichterle and Stoy developed a method of using sulfuric acid to treat acrylonitrile tubes, to make the surfaces of the tubes more slippery and wettable, in the hope that such tubes would be medically useful. However, none of the Examples described any actual medical testing or use; instead, all examples and data were limited to methods of manufacturing and then chemically treating the tubes.

A crucial difference must be recognized between cartilage implants, versus catheters and cannulas. Because of their nature and use, catheters or cannulas do not need to withstand any substantial or repeated rubbing, abrasion, or other stresses. Instead, they are usually inserted while a patient is fully or partially anesthetized, and then used to drain seepage or urine, usually for only a few hours or days. While in place, they're treated carefully and gently, to avoid injuring or irritating the surrounding tissue. Those types of tubes do not need, and do not have, the levels of strength, toughness, and durability that will be required of a cartilage-replacing implant that must withstand compression, wear, and abrasion over a span of decades.

It must also be recognized that the typical and expected effects of treating a solid surface with sulfuric acid involve etching, pitting, and other corrosive alterations. Except for certain specialized treatments designed to remove "scale" from steel or other metals, a treatment using sulfuric acid (or any other strong acid) almost always renders a treated surface rougher, rather than smoother. Therefore, there was no reason to expect or predict that sulfuric acid treatment of the surface of a hydrogel would provide a better surface for a cartilage-replacing implant. Even if a surface had improved "wetability" after a treatment, the risks of pitting, etching, and corrosion (which would make no difference in a catheter or cannula tube, but which would be crucial in an articulating cartilage surface) were regarded as very high.

Despite those concerns, the Applicant decided to try sulfuric acid treatments, to see what effects they might have on the polymers he was testing at the time, which were polyacrylonitrile (PAN), polyvinyl alcohol (PVA), and PVA/PVP copolymers that also contained poly-vinyl-pyrrolidone. The tribometer he was using can test six samples of material during each test run, with each sample isolated in its own shallow dish. Not all of the test runs being performed used all six positions available in the machine, so the Applicant decided to use the vacant positions to test samples that had been treated with sulfuric acid, using concentrations, temperatures, and incubation periods as described in US patent 4,183,884. The Applicant obtained the surface-treated samples from Hymedix (Princeton, New Jersey). That company is no longer in existence, but a key individual George Stoy (one of the sons of Artur Stoy, who did the earlier work with PAN) now works with PragTech Inc. (Flemington, New Jersey). It is believed that those materials (prior to the sulfuric acid treatment, which was carried out in the Applicant's labs) were made by methods such as described in US patent 6,593,451 (Stoy).

To the Applicant's surprise, the results of actual wear tests, on sulfur-treated PAN polymers, were quite good. Those results are described in the Examples, below. Other results from PVA or from PVA/PVP copolymers were less impressive, and by the time those results became available, it became clear that PAN was clearly a more promising and durable material than PVA, or PVA/PVP copolymers.

The tests conducted to date have focused on wetness and "feel" tests (using the fingertips), and on the types of long-term wear tests that can be carried out on a tribometer. The results of tests done to date indicate the following:
(i) PVA or PAN hydrogels can be rendered substantially more lubricious (this term implies a combination of wet and slippery), if they are treated by a sulfur-donating reagent, such as diluted sulfuric acid;
(ii) either PVA or PAN polymers will have their wear rates reduced, and their useful lives extended, in tribometer-type testing, if suitably treated by a sulfur-donating reagent, using parameters such as described in the Examples; and,
(iii) PAN polymers appear to be stronger and tougher, and less subject to wear and abrasion, than PVA polymers, when created and used in the manners described herein.

Because sulfur has various oxidation or valence states (as reflected in terms such as sulfates, sulfites, sulfides, sulfones, etc.), processes or reactions that add sulfur atoms to a hydrogel surface are referred to herein as "sulfuration". Since some candidate donor compounds contain sulfonic acid groups (R-SO₃H), the common term "sulfonation" can be used; however, it should be recognized that other reagents may be useful that do not have sulfonic acid groups, such as sodium sulfite or bisulfate, sulfur dioxide or trioxide, a mixture called oleum (SO₃ dissolved in sulfuric acid), and other reagents known to those skilled in sulfur chemistry (see, e.g., E.E. Gilbert, Sulfonation and Related Reactions (Interscience Publishers, New York, 1965)).

Any group that contains at least one sulfur atom and at least one oxygen atom can be referred to as a sulfate group; accordingly, many but not all sulfur donors will contribute sulfate groups to hydrogel surfaces. Terms such as moiety or end group imply that an atom or cluster of atoms is located at the end of a chain or side chain. Terms such as crosslink or bridge usually imply that an atom or cluster of atoms is (or will be, after a reaction occurs) located within a chain of some sort, and will not be located at the end of a chain.

As known to organic chemists, bonds formed directly between sulfur and carbon (or between sulfur and other non-oxygen atoms, such as nitrogen or phosphorus) have different bond strengths (and "breakability") than bonds with an oxygen atom between the sulfur and the carbon, nitrogen, etc. As a general rule, sulfur donors that create carbon-oxygen-sulfur linkages will need to be scrutinized carefully, since those indirect linkages are likely to be at greater risk of being broken (especially when contacted by biological fluids that contain esterases and other enzymes) than other sulfur-containing bond structures that can be created by skilled chemists.

Since sulfuric acid (H₂SO₄, or SO₄⁻² in ionic form) contains a sulfur atom that is completely surrounded by four oxygen atoms, in most cases it will create carbon-oxygen-sulfur bonds, when used to treat an organic polymer. Accordingly, now that it has been discovered that sulfuration of (and negative charge densities on) hydrogel surfaces can render hydrogel surfaces more lubricious and durable, this approach to chemically treating a hydrogel surface is likely to enable even stronger and more durable hydrogels, if other donor reagents are used that will not create indirect linkages (with oxygen atoms between carbon and sulfur atom), and instead will directly bond a sulfur or other electronegative atom to a carbon, nitrogen, or other selected atom or group in the polymer being treated. Similarly, hydrogels containing direct carbon-sulfur bonds can also be created by using sulfur-containing monomer reagents to form a hydrogel polymer; however, this will require careful evaluation of whether only the surface layers should include sulfur atoms, or whether sulfur atoms can be incorporated throughout the entire polymer without reducing strength, durability, or other desired traits.

Some sulfur-donating reagents (and some types of monomer reagents with sulfur-containing side chains or groups) can create crosslinking bonds or bridges that will connect different polymeric chains or other molecular structures. Such reagents merit evaluation, because higher numbers of crosslinking bonds, in a polymeric material, can impart greater strength, toughness, and durability to a polymer. Crosslinking reactions are widely used to give greater strength and durability to various materials. As an illustration, most "tanning" chemicals that process animal skins into tough and durable leather do their work by creating crosslinking bonds between collagen fibers in the animal skins.

Accordingly, experts in this branch of chemistry will recognize the ability of (i) polymerizable monomers containing side chains with sulfur or other electronegative atoms, and/or (ii) hydrogel-treating reagents that can donate sulfur or other electronegative atoms, to create hydrogel surfaces with greater lubricity and durability, which in many cases will include hydrogel surfaces having increased numbers of crosslinking bonds.

If desired, electronegative surface-treating agents that do not contain sulfur can be evaluated for use herein. For example, reagents that substitute halogen atoms for hydrogen protons, in a polymer, offer good candidates. The bonds between hydrogen protons and carbon atoms in a polymer are not strong, and replacement of hydrogen by other atoms or moieties can often lead to stronger, more durable polymers. Especially promising hydrogels are those in which some or all of the hydrogen atoms are replaced by fluorine. This principle is described in US patents 4,621,107 and 4,900,793 (Lagow et al), which disclose "perfluorinated" elastomers in which essentially all hydrogen atoms are replaced by fluorine. Fluorinated and perfluorinated polymers have better resistance to corrosion and wear than conventional polymers; accordingly, fluorinating agents, and prepolymer reagents that contain fluorine instead of hydrogen or chlorine in at least some locations, can be evaluated for use as described herein, and offer promising candidates for creating even stronger and more durable hydrogel compounds.

Alternately or additionally, monomers that can incorporate (or treating agents that can donate) non-sulfurous bivalent or multivalent electronegative atoms (such as nitrogen or phosphorus) into a treated polymeric surface can be evaluated for use herein to impart negative charges to a hydrogel surface, and/or to provide higher strength to a hydrogel surface due to crosslinking or other effects.

The importance of using electronegative atoms to create negative charges in the surface of a hydrogel is discussed in more detail below.

### NEGATIVE CHARGE DENSITIES ON CARTILAGE AND HYDROGELS

An important factor that must be taken into account, in evaluating the effects of electronegative atoms in a hydrogel surface, involves the fact that natural cartilage surfaces have negative electrical charges. As described in reports such as Maroudas 1979, the "fixed charge density" (FCD) of human cartilage ranges from about -50 to -250 millimolar (mM), depending on the age of the person, the location of the cartilage, and the status and condition of the cartilage.

When expressed in millimolar terms, negative charge density can be measured by using a vibratome to cut cartilage into very thin slices (such as 0.2 mm thick), then weighing each layer, and then equilibrating that known weight of cartilage with a known concentration of sodium ions (Na⁺) in a low-concentration saline solution. The amount of Na⁺ ions that are absorbed by the cartilage, in the process of taking it to an electrically neutral and uncharged state, is used to calculate "fixed charge density". This density is then adjusted to a standard wet weight for cartilage, assuming certain equilibrium values as described in Maroudas 1979.

Other ways of measuring FCD values have also been developed, including ways that express charge density in milli-equivalent (mEq) rather than millimolar levels, which can indicate the concentrations of proteoglycans in cartilage. Mow and Hayes 1991 reported that cartilage usually ranges from 0.05 to 0.3 mEq per gram of wet weight of cartilage tissue. Other articles that discuss the negative charge density of cartilage include Maroudas et al 1969, Stanescu et al 1982 and 1986, Van Damme et al 1992, Buckwalter et al 2000, and Maniwa et al 2001. All of these sources report that cartilage has a negative electrical charge, believed to be caused mainly by sulfate and carboxyl groups on chondroitin sulfate chains, sulfate groups on keratan sulfate chains, and carboxyl groups on hyaluronate chains (hydroxy groups on collagen fibers may also contribute somewhat to the negative charges on cartilage). Several of these articles also indicate that a decrease in charge density indicates that cartilage is suffering from a problem which, if not corrected, may lead to a breakdown and loss of the cartilage.

It is presumed and believed that negative surface charges help cartilage layers in synovial joints interact usefully with certain positively-charged components of synovial fluids. In particular, it is believed that the "heads" of certain synovial fluid components called "lubricin" have a localized or net positive charge. As described and illustrated in US patent 6,530,956 (by the same Applicant herein), lubricin molecules form molecular complexes with surface-active phospholipid (SAPL) molecules. These two types of molecules alternate back and forth between free (unassociated) forms, and complexes that are held together by ionic attraction. The complexes are assumed to form under resting conditions, when no compressive or shear forces are being exerted on a joint. Subsequently, when a joint is flexed and moved (especially under compression, as occurs in a knee when a person begins walking), lubricin/SAPL complexes can be separated, without damaging either component, in a manner comparable to pulling apart two relatively weak magnets. The separated components will eventually recombine, presumably after the activity ends and the joint returns to a resting state, thereby reforming new complexes, which will be ready to repeat the cycle when activity begins again.

Accordingly, it is believed that a negative charge density, on a cartilage surface, can help promote the proper formation, alignment, and activity of lubricin-SAPL complexes, by helping to ensure that the "heads" of lubricin molecules will line up in the desired manner, in a joint that is at rest.

Unless and until experimental evidence indicates otherwise, it is presumed and believed that the negative electrical charge density on a synthetic hydrogel implant should emulate the negative electrical charge density on natural, healthy cartilage. Accordingly, unless experimental evidence indicates otherwise, the charge density on a hydrogel implant surface should have a fixed charge density within a range of about -50 to about -250 mM, measured by sodium equilibration.

The charge density on a synthetic polymer can be altered and controlled to any level of interest, by methods known to those skilled in the art. As one example, if a lower charge density is desired, a polymer surface can be treated with a lower concentration of a sulfur-donating or similar reagent, and the treatment period can be reduced. Alternately, if a higher charge density is desired, a polymer surface can be treated with longer treatment periods, by repetitive treatments, by a series of treatments using progressively stronger reagents, and/or by using pre-polymeric reagents with higher quantities of electronegative groups.

Using the machinery and methods disclosed in the Examples below or otherwise known to those skilled in the art, samples of such treated polymer surfaces having a range of charge densities can be tested to evaluate their wear factors, their coefficients of friction, and any other parameter of interest. By means of such tests, which will require only routine experimentation, an optimal charge density can be determined for any type of polymer of interest (such as polyacrylonitriles or polyurethanes that have been sulfonated, phosphorylated, or otherwise treated).

The optimization of preferred treatment steps (which will include testing a range of temperatures, concentrations, incubation periods, and other conditions for carrying out a treatment, after a set of reagents has been chosen) for creating an improved surface layer will depend on the particular type of polymer being treated, and the particular sulfur-donating or other chemical reagent chosen for the treatment. However, the testing of such treatment parameters is well within the skill of the art, and can be carried out using no more than routine experimentation, once the goal of a surface treatment is adequately understood. This is especially true since any such surface treatment can be evaluated by comparing its results to a sulfonation treatment as disclosed herein, which can be regarded as providing a "benchmark" level of improvement in surface layers for hydrogel cartilage implants.

Examples 3 and 4, below, disclose preferred methods of testing candidate surface-treated polymers, to evaluate the effects of a candidate chemical treatment on (i) the strength and durability of the resulting treated polymer, and (ii) the ability of a treated polymer to interact properly with synovial fluid.

On the subject of testing, it should be noted that the Applicants herein tested various candidate polymers, using two different fluids. One of those fluids is actual synovial fluid, from cows. Although synovial fluid assertedly is commercially available, the only available preparation was inordinately expensive, so the Applicant and an assistant obtained it by visiting a slaughterhouse, and using a syringe to aspirate fluid from the ankle joints of freshly-killed carcasses. Roughly 5 ml could be obtained from a typical ankle joint. Before use, the fluid was filtered twice through a 20 micron filter, then 0.2% w/v sodium azide (a preservative) was added with stirring. It was filtered again, then it was frozen at -20°C until use.

Synovial fluid is not widely used by researchers and companies that test candidate materials for replacing cartilage, for two reasons. First, it is quite expensive. Second, it makes reliable testing more time-consuming, since it provides extremely good lubrication that tends to mask and obscure the differences in performance between candidate materials being compared against each other. To avoid those problems, most researchers and companies use bovine blood serum as a lubricating liquid, in most such tests.

Accordingly, for comparative purposes, the Applicant ran various tests, using bovine blood serum in some tests, and synovial fluid in other tests. The results, provided in Table 1, indicated that: (i) polyacrylonitrile (PAN) polymers as well as PVA/PVP polymers both performed better, if their surfaces were sulfonated; and, (ii) PAN polymers tended to perform better than PVA polymers or PVA/PVP copolymers. Furthermore, tests using synovial fluid generated more consistent results, while tests using blood serum tended to give more scattered results.

### ELIMINATION OF NON-PLANAR INTERFACE LAYER;

### REPLACEMENT WITH MESH AND ANCHORING LAYER GRADIENTS

Two prior utility patent applications filed by the same Applicant herein (serial numbers 10/011,933, filed Nov. 30, 2001 and published as US application 2002/173,855, and serial number 10/071,930, filed Feb. 8, 2002 and published as US application 2002/183,845) described a "multi-perforated non-planar interface" layer (also referred to as a "perforated waffle" layer) as an important part of a flexible hydrogel implant of the type described herein, for replacing hyaline cartilage. Those disclosures were based on computer modeling, which showed that a relatively thin layer of a molded plastic or similar material, having numerous raised bumps, squares, or other surface protrusions, and having the facets of the bumps or squares perforated by holes, could provide an improved, stronger, more secure interface between a layer of soft hydrogel material, and a layer of harder material suited for anchoring to a hard bone.

Briefly, if the interface between two different materials having very different hardness and stiffness levels is merely flat and planar, then any shear forces that seek to push and slide the soft coating layer off of the hard support layer will effectively "focus on" the flat and planar boundary between the soft and hard materials, and will pose a greater risk of damaging the implant.

That underlying factor remains true and valid; however, the Applicant realized that, in a flexible implant that is only a few millimeters thick, a molded non-planar plastic layer, with holes cut through the uppermost surfaces of protrusions that rise above the baseline plane of the plastic layer, can pose a serious risk of cutting through the hydrogel material that sits directly above one of those plastic protrusions. This risk becomes even more serious if a person with a knee or hip implant suffers a fall or other accident that causes high instantaneous (peak) compression. Although steps can be taken to reduce this risk (such as by making the layer of plastic material relatively thin and flexible, especially on the tops of the protrusions), such steps will reduce the strength and reinforcing benefits of the molded reinforcing layer. Even if such steps are taken, some risk of cutting through an overlying layer of hydrogel, in a manner that might puncture or lacerate the smooth surface of the implant, will remain, if a molded plastic interface with holes cut through it is embedded in a hydrogel implant that is only a few millimeters thick.

Without taking any position on whether such risk levels could be tolerated in at least some classes of implants, the Applicant began to consider how the molded perforated plastic layer could be eliminated, and replaced by a different type of reinforcing component or layer that would not pose those risks.

A preferred approach resides in the use of gradients, to prevent the formation or existence of a stark or sharp boundary between a hard anchoring material, and a hydrogel coating layer. Such gradients can be created and provided, in both: (i) the flexible fibrous mesh that will reinforce the layer of hydrogel material, and (ii) the porous anchoring material that will press against the prepared bone surface, and promote tissue ingrowth.

This does not assert that such gradients are necessary and essential to render such implants fully functional. Instead, it may be that a single consistent type and density of reinforcing mesh can provide fully sufficient reinforcement, without the use of such gradients. However, gradients can offer certain advantages (such as lower impedance to water permeability through the outermost surfaces of a gel layer) which can enable an implant to more closely simulate native and natural cartilage near the articulating surface, while having improved strength, toughness, and durability due to higher levels of reinforcement in the lower layers. Therefore, gradients in the reinforcing materials merit testing and evaluation as this invention progresses through the mechanical and then animal tests that will be required before human clinical trials can commence.

With regard to a flexible fibrous mesh that will reinforce the hydrogel material in an implant, gradients can be created in any of several ways, and the selection of preferred methods and fiber types for providing such a gradient, in a reinforcing mesh, will depend on the methods and fiber type(s) being used to create the mesh. For example, it is believed that in a "Techniweave" type of operation (as developed and commercialized by a company called Techniweave (www.techniweave.com, a subsidiary of Albany International), a first type of fiber having a first selected thickness and density can be used to form all or the majority of a first layer or zone, while a second type of fiber having a second selected thickness and density can be used to form a second layer or zone (or the majority thereof).

Alternately, if a needle-punching or similar operation is used, a first layer of fibers having a first selected thickness can be laid onto a conveyor belt at a first selected density, and a second layer of fibers having a second selected thickness can be laid onto a conveyor belt at a second selected density. Subsequently, the bi-layer mat can be compressed and needle-punched or otherwise treated.

Alternately or additionally, the use of continuous fibers in one layer of a mesh, and chopped fibers in a different layer of a mesh, can provide mechanical and performance gradients of the type anticipated herein.

These are examples of approaches known to those who are skilled in fabric or carpet manufacturing, and in the design and operation of fiber-handling and carpet manufacturing machinery. In addition to the Techni-Weave process mentioned above, anyone interested in how three-dimensional meshes can be manufactured from fibers can review published sources describing how various types of tufting structures are used to make carpet. An introduction to carpet types and weaves, showing cross-sectional drawings of various types of continuous loop and cut-pile arrangements, is available from the University of Nebraska website, at http://ianrpubs.unl.edu/homefurnish/g1316.htm. Information on how various machines and methods are used to create those structures is well-known within the carpet industry, and those types of machines and methods can be adapted to create fiber meshes with reduced dimensions, for use in cartilage-replacing implants. If desired, various such methods and machines also can be adapted in ways that will create density and porosity gradients in the resulting meshes.

Porosity gradients in harder materials, for the anchoring surface of an implant (i.e., the surface that will press against a bone surface from which the cartilage has been removed) can also be created by means such as described above, or by various other means known to those skilled in the art. As one example, granular particles that will dissolve in a selected liquid (such as grains of salt or sugar, which will dissolve in water), and which can have varying diameters if desired, can be mixed with pre-polymeric liquids or granules, at concentrations or ratios that will vary depending on their depth in a molding cavity. In this approach, a controlled concentration of soluble grains can be positioned in the bottom of a mold, to create an anchoring surface, while different concentrations of soluble grains can be positioned in higher layers of the mold. After the pre-polymer material is cured to form a very strong polymer, a solvent is used to remove the grains, leaving behind a porosity gradient. A liquid pre-polymer that will form a hydrogel is then permeated through the harder porous polymer, and cured to form the hydrogel, which will be reinforced by the stronger polymer.

As another example of an approach that can be used, calcium phosphate mixtures similar to hydroxyapatite, the crystalline mineral that forms hard bone, can be coated (such as by "sputter-coating", which is more precise and even than liquid plating methods) onto various types of porous materials (such as thick but flexible screen meshes made of very thin metal wires) that have been provided in advance with density gradients.

These are examples, for illustrative purposes, and experts who specialize in designing and fabricating these types of materials will recognize other approaches as well.

Accordingly, density and stiffness gradients can be created using any of various means, in both (i) a flexible mesh of the type that will be used to reinforce a hydrogel material, and (ii) a porous material that will promote tissue ingrowth, in an anchoring layer.

By proper selection and use of gradients in both types of materials, a gradual and non-planar transition zone can be created between a soft hydrogel material and a harder anchoring layer, in an implant as described herein. Such transition zones can eliminate the need for a non-planar interface layer made of molded and perforated plastic, which could pose a risk of lacerating or puncturing the hydrogel layer in a person who suffers a fall or other accident.

If desired, the anchoring layer and the reinforcing mesh in a hydrogel can be created from a single type of material, if the material is provided with adequate gradients in density, thicknesses of the strands, or other parameters. As one example, a highly flexible reinforcing mesh that would pose no danger of lacerating or puncturing a hydrogel can be made of wire (or polymer) strands that are so thin they are difficult to see with the naked eye. As these super-thin strands increase in thickness, they become stiffer as a function of cross-sectional area (i.e., as a squared function of the radius or diameter). At one or more transition zones, the strands can be made thicker, and can be woven together more densely. By the time the bottom of the implant is reached, the stranded mesh can be sufficiently thick and dense to make it difficult to flex and bend without substantial effort. This type of mesh can provide an anchoring layer, while the thinner strands will be much more flexible and will reinforce the hydrogel, close to the articulating surface.

### ANCHORING PEGS AND RECEPTACLES

Any suitable method can be used to anchor an implant as described herein to a prepared bone surface. After studying various factors, issues, and items of prior art, the Applicant has identified and adapted a certain type of anchoring system that is believed, for reasons discussed below, to offer a preferred candidate system for early evaluation.

The anchoring system that is disclosed and preferred herein, as illustrated in FIGS. 1 and 2, uses anchoring pegs 170 that are designed to lock into place when pressed into locking receptacles 180. Receptacles 180 are designed to be inserted and affixed (such as by cementing, screws, press-fitting of a ridged shape, etc., or by providing the entire receptacle with an externally-threaded surface shape) into recesses that will be drilled or otherwise created in the hard bone that will support the implant, prior to insertion of the implant 100 into the joint that is being repaired.

To understand the merits and limitations of this anchoring system, it should be compared to other anchoring systems that have been recently proposed in the art, such as the systems disclosed in US patents 6,626,945 and 6,632,246 (both by Simon et al 2003), briefly discussed in the Background section herein. As illustrated in Figure 10 in each of those patents, and as described under the "Method 1" through "Method 4" subheadings that precede the Examples in both patents, the "peg" components of Simon et al are either machined or molded from a "biocompatible polyurethane" with a hardness similar to subchondral bone. They apparently must be inserted, presumably by gentle-to-firm tapping and force-fitting, directly into suitably sized holes that have been drilled, grinded, or otherwise machined in the patient's bone, apparently without the use of any surrounding cement or other material. Once in place, the ridges around the surfaces of the pegs presumably will keep them in place, embedded in the bone, presumably for years or even decades. While the surgery is still in progress, a "flowable polymer" having a different hardness is spread across the surface that is being repaired, and it assertedly will stick to the exposed tips or shoulders of the pegs, in an assertedly satisfactory and durable manner.

The system developed and disclosed by the Applicant herein works in a different manner. During a first preparative step, the placements of the locking receptacles 180 will be established, with the help of one or more templates, tool guides, computerized navigational equipment, or comparable devices that are already known in the art, or that can be developed by experts working on this particular step in the surgical procedure. The appropriate holes can be drilled, machined, or otherwise created in the bone, and the receptacles can be emplaced therein, with the assistance (if desired) of cement, anchoring pins, or other means. As just one example, a cement can be used that will not harden for more than an hour, to allow the pegs of the implant to be inserted into the receptacles before the cement fully hardens and establishes their exact final alignment.

If desired, means can be provided to allow the pegs to be removed from the receptacles, if and when the need arises, by means that may include deliberately destroying the remainder of the implant (allowing it to be completely replaced by a new implant, without requiring any alteration of any sort in the anchoring receptacles that remain embedded in the bone). Such removal means may include, for example, a rotatable mechanism (with screw-type threads, a so-called "bayonet" fitting, etc.) that can be accessed only from the top of the peg, by going through the hydrogel layer of the implant. Alternate removal means might include, for example, inserting a shim-type device into a slot between two halves of a peg, to expand the peg segments in a way that locks them into place, requiring the shim to be removed before the peg can be removed.

Still other means that can reliably prevent accidental disengagement, but that can be removed by a surgeon if the hydrogel portion of an implant must be replaced, can be developed, if desired. For example, rather than inserting a shim-type wedge into a split peg, a paste-type adhesive that will cure into a hardened material can be inserted into the receptacle, just before the pegs are inserted, in a quantity that will ensure that the gaps in the split pegs (as illustrated in FIG. 2) will be filled by the soft material. The paste will then harden after the surgery is completed, while the joint is still immobilized, in a manner that effectively locks the pegs in place.

Another factor that should be noted is that the outer diameter (and circumference) of a receptacle, such as illustrated, is substantially larger than the diameter (and circumference) of a peg. This allows wider receptacles to provide larger and stronger "gripping surface" areas than thin pegs, which should be slender enough to allow a flexible implant to be at least partially rolled up, during insertion, for arthroscopic insertion.

If this approach is used, it also is possible to couple the anchoring pegs to an implant by flexible means, to allow better folding and easier arthroscopic insertion, without sacrificing strength and reliability. As one example, if strong polymers having sufficient tensile strength are used, each peg 170 can be attached to the bone anchoring layer 150 by means of a hinged device or flap, to allow the peg to be rotated into a flatter position, parallel or at least closer to anchoring layer 150, during insertion. If this design is used, hinges or flaps on different pegs on the implant 100 can be provided with different placements and directional orientations, if desired, so that all of the pegs will reinforce each other and, acting together, prevent any transverse displacement of the anchoring layer in any one direction, during the initial recovery stage while tissue is growing into the porous anchoring layer.

Alternately, if desired, anchoring layer 150 can be provided with smaller "peg attachment devices", for easier and more compact arthroscopic insertion, and the full-length pegs 170 can be affixed, one at a time, to the smaller attachment devices, after the peg receptacles have been emplaced in the underlying bone, and after the implant 100 has been inserted into the joint and is being prepared to be permanently locked in place by inserting the affixed pegs into the receptacles.

This implantation and anchoring system is believed to provide various advantages over the prior system described and illustrated in US application 2002/173855. In that prior embodiment, it was suggested that the upper portion of the assembly (including the hydrogel layer and the "perforated waffle" layer) could be affixed to an "anchoring grid", indicated by callout number 200 of FIG. 1 in US application 2002/173855. However, several potential problems with that approach became apparent, during additional efforts that followed the filing of that earlier application. Those potential problems included:
(1) because of its original design, as illustrated in US application 2002/173855, it might be difficult to adapt that type of grid for solid and secure placement on certain types of implants, such as for replacing femoral runners, because of the length and the extensive curvature of a femoral runner;
(2) the use of an anchoring grid, and the need to develop a method for securing the remainder of the implant to the grid in a manner that would endure for decades in a joint that is subjected to frequent stresses and loadings, might pose a risk of creating one or more types of irregularities (such as humps, bumps, ridges, etc.) on the hydrogel surface of the implant; and, any such irregularities, even if only minor, might lead to serious damage to any or all hydrogel implants in a joint, over a span of years; and
(3) as a general rule, any act of assembling different components together into a unit, during an implantation operation that requires *"in situ"* assembly of different parts inside a joint such as a knee, creates some unavoidable level of risk that the parts that were assembled and attached to each other, during the operation, might eventually work their way loose or become separated and undone, after years of repeated loadings and stresses. Therefore, if steps can be taken to avoid having to assemble such components during surgery, the risk that such components might loosen or become detached, years later, can be minimized.

Accordingly, the Applicant continued to work on the design of the anchoring system, and developed the design disclosed above and in the drawings, which is believed to offer various advantages for at least some and probably most candidate uses in load-bearing joints.

### SURFACE TREATING OF WEDGE-SHAPED MENISCAL OR LABRAL IMPLANTS

In addition to creating "condylar" implants, which will be anchored to hard bone surfaces, surface treatments as disclosed herein (using agents such as dilute sulfuric acid) can be used to created improved implants for replacing meniscal cartilage (in knee joints) and labrum cartilage (in hip and shoulder joints). Rather than covering a rounded bone surface, these specialized arc-shaped cartilage segments have wedge-like cross-sections. They are anchored to soft tissue around their periphery, and to limited areas of bone (such as the tibial spine) via fibrous extensions that emerge from their "arcuate tips".

The chemical or other steps for treating the surface of a meniscal or labral implant, to give it more lubricious upper and/or lower surfaces and a negative charge density that emulates natural cartilage, can be adapted directly from the methods disclosed above, for treating condylar implants.

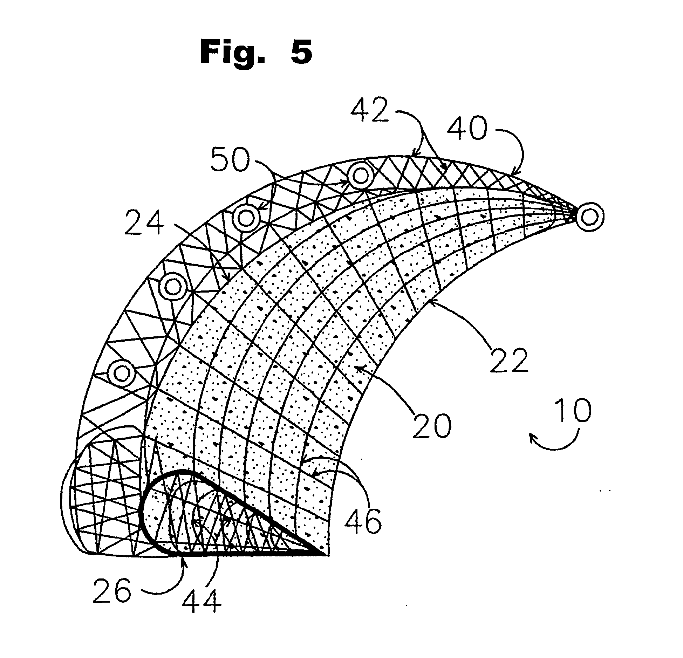

FIG. 5 illustrates a meniscal implant 10, containing a hydrogel polymer 20 indicated by stippling. Meniscal implant 10 has an arc-like or crescent shape, comparable to a curved claw, with an inner surface or edge 22, and a peripheral or rim surface 24. The treated surface layer (or "skin") of the meniscal implant is shown by a heavy line, indicated by callout number 26 in the cutaway segment at the bottom of the drawing. Reinforcing mesh 40 comprises two classes of long fibers. Peripheral (or anchoring) strands 42 extend out of the implant 10, around the rim surface 24, which does not need to be smooth because it is not an articulating surface; five anchoring reinforcements 50, located around the periphery of the mesh 40, are also shown. Penetrating mesh strands 44 enter and pass through the hydrogel polymer 20. Due to the woven, knitted, or other three-dimensional structure of the mesh, a single long strand in mesh 40 might provide a peripheral/anchoring strand 42 at one location, and a penetrating strand 44 at another location.

Fibrous strands 46 are visible in the perspective view portion of the drawing, because the thin layer of hydrogel material that covers fibrous strands 46 is essentially clear and transparent, due to its high water content. However, those fibers preferably should not be exposed on either of the upper or lower articulating surfaces of implant 10, because their presence on an articulating surface would generate elevated levels or risks of roughness and abrasion. Accordingly, strands 46 are shown for illustration only, to indicate that the mesh extends throughout and reinforced essentially the entire hydrogel component, other than the treated surface layer 26.

### EXAMPLES

### EXAMPLE 1: PVA AND PVA/PVP SAMPLES

Granular PVA (grade 71-30, with an average molecular weight of about 140 kilodalton) was supplied at no cost by DuPont. PVP (average molecular weight about 40 kd) was obtained from Sigma Chemical. When PVA/PVP copolymers were tested, they contained a ratio of 99 % PVA and 1% PVP, by weight. In either case, a total polymer weight of 10% w/v in distilled and deionized water was used. The mixture was stirred for 20 minutes, by which time the solution appeared to be completely uniform and consistent. It was heated to 85°C overnight, then cooled to room temperature, and stirred again for 20 minutes.

An aliquot of this solution was poured into a shallow flat mold, which was then kept in a warm ventilated incubator at 37°C until essentially all water had been removed, leaving a polymeric sheet with a thickness of about 1.75 to 2 mm. This usually took about 4 to 5 days.

A punch was used to remove circular samples, usually with 0.67, 1.5, or 1.625 inch diameters, depending on the tests that were planned. Before testing, these samples were fully hydrated and swelled in aqueous phosphate-buffered saline (PBS) solution, for 1.5 to 2 days.

Several PVA/PVP samples were also formed using an alternate procedure. After overnight incubation at 85°C as described above, a PVA/PVP solution was cooled to room temperature and then poured between glass slides that were separated at their ends by spacers with equal thicknesses. This established a consistent thickness. The samples were then frozen at -20°C for 20 hours, then thawed to room temperature for 4 hours. This cycle was repeated 6 times, to obtain a hydrogel sheet, which was then cut into samples for testing, using a punch. The polymers formed by this method had more consistent and uniform thicknesses; however, they were not as strong and durable as the polymers formed by complete dehydration over a period of several days.

A third method of preparation was also tested, in which a combination of partial dehydration and freeze-thawing was used. This method involved pouring a quantity of the polymeric liquid into a shallow mold, to a level indicated by a mark at a fixed height, and then dehydrating the polymer at 37°C until the level of the remaining material had decreased to the height of a lower mark. This partial dehydration was then followed by several cycles of freeze-thawing. The resulting materials were shown to have intermediate levels of strength and durability, between the fully dehydrated samples and the freeze-thawed samples, and this method offered good control over the thickness of the resulting hydrogel.

The steps that were used to sulfonate any of the above-described PVA/PVP samples were adapted from the procedures disclosed in US patent 4,183,884 (Wichterle and Stoy, 1980), as follows. A solution of 60% sulfuric acid was prepared, and heated to 50°C with stirring. If both surfaces of a PVA/PVP sample were to be sulfonated, the sample was dropped into the 60% sulfuric acid solution, for 2.5 minutes. The sample was then removed, rinsed with distilled water, dipped briefly into a mild alkaline (NH₄OH) solution to fully quench any remaining acid, and then rinsed again.

Problems were encountered when samples treated on both sides were tested in the tribometer, since high levels of slipperiness on both sides of a sample interfered with secure affixation within the machine. Therefore, subsequent tests used samples that were sulfonated on only one side. This can be done in various manners, such as by placing a custom-cut sheet of polymer in the bottom of a shallow dish or tray, in a manner that causes the edges of the polymer sheet to form lips around the sides of the dish. A quantity of 60 % sulfuric acid is then poured on top of the polymer layer, in a quantity that does not cause the acidic solution to spill over the edges of the polymeric sheet. The acid solution is poured off after 2.5 minutes, then the sample is rinsed, quenched, and rinsed again as described above. Samples for testing are then cut from the interior region of the sheet that was treated.

By the time the Applicant and his assistant were ready to run a complete set of tests on sulfonated PVA/PVP, so that it could be compared to non-sulfonated PVA/PVP, it had become clear to them that polyacrylonitrile polymers were providing stronger and more durable hydrogels, than PVA or PVA/PVP polymers. Therefore, a full set of comparative tests (as described below) on sulfonated PVA/PVP was not carried out.

### EXAMPLE 2: POLYACRYLONITRILE SAMPLES

Sample sheets of polyacrylonitrile, 2.55 to 2.6 mm thick, were provided by the PragTech company (Flemington, New Jersey). These sheets were of a type designated as "Qpan" by Pragtech. The exact details of the process use to manufacture the "Qpan" class of PAN are proprietary, and may be covered by one or more currently pending patent applications (including US applications 09/383,020 and 10/193,578, both by Stoy et al and accessible on the U.S. Patent Office website). Methods for manufacturing polyacrylonitrile are disclosed in various patents that can be located by searching for "Stoy" as the inventor, in the U.S. patent database (www.uspto.gov). Such US patents range from 4,107,121 ("Ionogenic hydrophilic water-insoluble gels from partially hydrolyzed acrylonitrile polymers...") to 6,593,451 ("Method of processing polyacrylonitrile"), and include 14 additional patents in between those two. US patents 3,895,169 and 4,183,884 also deserve mention, because they relate to surface-layer sulfonation of PAN polymers.

Circular samples of the Qpan polymer were cut from the Pragtech sheets by means of a punch. These samples were sulfonated by the same procedures disclosed above for the PVA/PVP polymers.

### EXAMPLE 3: STANDARDIZING TESTS ON TRIBOMETER

Before the tribometer (made by AMTI, www.amtiweb.com, and connected to a desktop computer using AMTI software) could be used for testing hydrogels, it had to be standardized, which is comparable to calibrating it. This is done using the procedures set forth in ASTM protocol F732 ("Standard Test Method for Wear Testing of Polymer Materials Used in Total Joint Prostheses").

Briefly, the tribometer machine is used to rub pins having smooth, flat-faced surfaces made of a known type of plastic, called "ultrahigh-molecular-weight polyethylene" (UHMWPE), against smooth disks made of a very hard cobalt-chromium alloy (supplied by Biomet Inc., www.biomet.com). Prior to the tests, the pins (having 0.5 inch diameters for the standardizing tests) were pre-soaked in distilled water for a month, to minimize fluid absorption during the test. A load of 253 newtons was applied to the pins, to generate an average contact stress of 3.54 megapascals (Mpa). The tribometer was programmed to move the table, which supported the discs, m a circular wear path having a 50 mm perimeter. The wear cycle frequency was 1 hertz (i.e., 1 cycle per second), giving a sliding velocity of 50 mm/s.

When a test is ready to begin, the pins are lowered onto the discs, until a known amount of force (expressed in newtons) is exerted on the pins. Based on the surface area of the pins, this generates a controllable amount of pressure (expressed in megapascals, mPa) on the UHMWPE surfaces at the bottoms of the pins. The relationship between force and pressure can be checked for accuracy by using pressure-sensitive film, such as Fuji "Pressurex" film.

Newborn bovine calf serum (ICN Biomedicals) was diluted to 50 % (by volume) with distilled water and used as the lubricant. As specified by the ASTM standards, the lubricant contained 0.2 % sodium azide, and 20 millimolar ethylene-diamine-tetraacetate (EDTA), as preservatives. The temperature of the lubricant was maintained at 37 ± 1°C throughout the test period, using a recirculating temperature control unit. The test was done for 2 million cycles, amounting to a wear path length of 100 km (about 62 miles).

After 2 million cycles, the UHMWPE plastic at the ends of the pins had an average weight loss of 24.57 mg. This converts into an average wear rate of 12.82 ± 1.33 mm³ per million cycles (mean ± standard deviation), and a calculated "wear factor" of 10.1 ± 1.05 x 10⁻⁷ mm³ per newton-meter.

These values correlate well and were close to the wear rates and wear factors of tests reported by other researchers, using UHMWPE. Accordingly, these standardizing tests confirmed that AMTI Tribometer was working and calibrated properly.

### EXAMPLE 4: WEAR TESTING OF HYDROGELS

Testing of hydrogels was conducted in either 100% fetal bovine serum, or 100% synovial fluid. Prior to usage in the wear tests, 0.2 % sodium azide was added as an antibacterial agent, and the lubricant was filtered twice through a 20 micron filter. The hydrogel samples, after being attached to the pins and the discs, were soaked in the lubricant for at least two hours before the start of any test. Temperatures of all lubricants were maintained at 37 ± 1°C throughout the test period.

The machine can test up to 6 samples at once, each using its own pin. The hydrogel samples affixed to the pins and the discs were taken from the same sheet of material, to ensure that they had the same thicknesses. An "upper" hydrogel sample is affixed to the bottom of each pin used, using a cyanoacrylate adhesive, while "lower" hydrogel samples were attached firmly to stainless steel disks (1.7 inch diameter) with the help of acrylic fixtures. These fixtures also provide a shallow tray, which holds lubricant at a depth that will cover and bathe both of the hydrogel samples throughout a test. As mentioned above, samples that were sulfonated were treated on only one side, to allow the untreated side to provide better adhesion to the pins.

When the machine and a set of samples were ready, a force level (usually ranging from 100 to 170 newtons) was chosen for that test. When a force of 150 newtons was applied to six pins, it generated an average contact pressure of 2.9 megapascals (Mpa), which falls within normal physiologic stress levels that are typical in an adult joint.

When the test begins, the platform that supports the stainless steel discs and the lower samples begins to move in a programmed motion. While the standardization tests used a circular motion, the wear-testing of hydrogels used a straight-line reciprocating motion, 30 mm in length. The cycle frequency was increased to 1.67 hertz, which maintained the sliding velocity at 50 mm/sec, which is the ASTM F732 standard recommendation. If a test continued for 1 million cycles, the total sliding distance was 30 kilometers (about 18 miles).

In a typical test, samples were tested for up to half a million cycles, with occasional visual inspections at intervals that depended on the amount of force being used for that test. For example, in an initial test of sulfonated PAN using synovial fluid as the lubricant, no visible wear was detected after 487,000 cycles, at a testing force of 100 newtons; therefore, new pieces of material were tested at increasing forces of 125 newtons, then 150 newtons, then 175 newtons. The testing at 175 newtons disclosed visible wear after 120,000 cycles.

When non-sulfonated PVA or PVA/PVP hydrogels were tested, using synovial fluid lubrication, visible wear was seen after only 4000 cycles, under an 80 newton load.

When sulfonated PVA or PVA/PVP hydrogels were tested, using synovial fluid lubrication, the samples showed significant visible wear after 2000 cycles at a 175 newton load.

When a non-sulfonated PAN sample was tested in synovial fluid, the hydrogel did not survive more than 10,000 cycles. This excessive wear was attributed to a higher friction coefficient for the non-sulfonated sample.

When sulfonated PAN was tested in synovial fluid, the gel survived 487,000 cycles at 100 newtons, with only one small scratch visible, apparently due to a tiny glue fragment that was exposed. Testing had to be stopped due to failure of the glue that held the gel to the base disc.

Subsequent testing of sulfonated PAN at loads of 125 newtons was terminated at 195,000 cycles, due to failure of the glue fixation. However, there was no visible wear on the articulating surface.

By the time these tests were finished, it was clear that: (i) polyacrylonitrile polymers provided more durable hydrogels than poly(vinyl alcohol) polymers; and (ii) sulfonation of the polymer surface increased the lubricity (slipperiness) of either type of polymer, and can provide a useful improvement for cartilage-replacing implants.

### EXAMPLE 5: COEFFICIENT OF FRICTION TESTS

In addition to the wear tests described above, coefficient of friction values were determined for a number of hydrogel samples. These values were measured while a sample articulated against the same type of material, using either bovine blood serum or bovine synovial fluid as the lubricant, and using various force and pressure loadings. Those values are provided in Table 1.

**TABLE 1**

| **COEFFICIENTS OF FRICTION FOR HYDROGEL SAMPLES** | | |
|---|---|---|
| **MATERIAL** | **LIQUID & LOADING** | **COEFF. FRICTION** |
| Non-Sulfonated PVA/PVP (99:1) | Blood serum, 80 newtons | 0.09 - 0.12 |
| Non-Sulfonated PVA/PVP (99:1) | Synovial, 80 newtons | 0.06 - 0.08 |
| Non-sulfonated polyacrylonitrile | Synovial, 175 newtons | 0.01 - 0.16 (average 0.07) |
| Sulfonated polyacrylonitrile | Synovial, 80 or 100 newtons | 0.01 - 0.03 |
| Sulfonated polyacrylonitrile | Synovial, 125 newtons | 0.04 - 0.05 |
| Sulfonated polyacrylonitrile | Synovial, 175 newtons | 0.02 - 0.11 (average 0.03) |

Thus, there have been shown and described new and useful implant devices for repairing or replacing damaged or diseased cartilage, and new methods for surgically implanting such devices. Although this invention has been exemplified for purposes of illustration and description by reference to certain specific embodiments, it will be apparent to those skilled in the art that various modifications, alterations, and equivalents of the illustrated examples are possible.

### REFERENCES

Ambrosio, L., et al, "Composite hydrogels for implants," Proc Inst Mech Eng [H] 212: 93-9 (1998)
Blue, M.A., et al, "In vivo results of hydrogel composite pericardial substitutes," ASAIO Trans 37: M152-3 (1991)
Buckwalter, J.A., et al, Orthopaedic Basic Science: Biology and Biomechanics of the Musculoskeletal System (2nd edition, American Academy of Orthopaedic Surgeons, 2000)
Corkhill, P.H., et al, "Synthetic hydrogels. VI. Hydrogel composites as wound dressings and implant materials," Biomaterials 10: 3-10 (1989)
Gorman, S.P., et al, "Characterization and assessment of a novel poly(ethylene oxide)/polyurethane composite hydrogel (Aquavene) as a urethral stent biomaterial, "J Biomed Mater Res 39: 642-9 (1998)
Maniwa, S., et al, "Alteration of collagen network and negative charge of particular cartilage surface in the early stage of experimental osteoarthritis, "Arch Orthop Trauma Surg. 121: 181-5 (2001)
Maroudas, A., et al, "The correlation of fixed negative charge with glycosaminoglycan content of human articular cartilage," Biochim Biophys Acta 177: 492-500 (1969)
Mow, V.C. and Hayes, W.C., Basic Orthopaedic Biomechanics (2nd edition, Lippincott-Raven, 1991)
Stanescu, R., et al, "The negative charge of articular cartilage surfaces: An electron microscopic study using cationized ferritin," J Bone Joint Surg Am 64: 388-98 (1982)
Stanescu, R., et al, "The negative charge of articular cartilage surfaces," Arthritis Rheum 29: 573 (1986)
Van Damme, M.P., "The measurement of negative charge content in cartilage using a colloid titration technique," Anal Biochem 204: 250-7 (1992)
Walker, A.S., et al, "Performance of a hydrogel composite pericardial substitute after long-term implant studies," ASAIO J 38: M550-4 (1992)
Wang, N., et al, "A heterogeneously structured composite based on poly(lactic-co-glycolic acid) microspheres and poly(vinyl alcohol) hydrogel nanoparticles for long-term protein drug delivery," Pharm Res 16: 1430-5 (1999)

## Claims

1. A surgical implant (100) for replacing cartilage in a mammalian joint, comprising a hydrogel material containing a synthetic polymer,
**characterized in that** said surgical implant (100) has at least one smooth and lubricious surface (112) with a negative electrical charge density that promotes lubricious interactions with mammalian synovial fluid.

2. The surgical implant (100) of Claim 1, wherein a flexible fibrous reinforcing matrix (122) is embedded within at least a portion of the hydrogel material.

3. A surgical implant (100) according to Claim 1 or Claim 2, wherein the smooth and lubricious surface (112) has a negative electrical charge density within a range of about -50 to about -250 millimolar, when measured by sodium equilibration.

4. A surgical implant (100) according to Claim 1 or Claim 2, wherein the smooth and lubricious surface (112) contains sulfur atoms.

5. A surgical implant (100) according to Claim 1 or Claim 2, wherein the smooth and lubricious surface (112) contains fluorine atoms.

6. A surgical implant (100) according to Claim 1 or Claim 2, wherein the smooth and lubricious surface (112) has been treated with a chemical reagent that creates crosslinking bonds between polymeric molecules.

7. A surgical implant (100) according to Claim 1 or Claim 2, wherein the implant has an anchoring surface provided with means for anchoring the implant to a prepared bone surface.

8. The surgical implant (100) of Claim 7, wherein the means for anchoring the implant to a prepared bone surface comprises pegs (170) that are designed to fit into accommodating receptacles (180) that can be inserted into a prepared bone surface prior to insertion of the surgical implant (100) into an articulating joint.

9. The surgical implant (100) of Claim 7, wherein at least a portion of the anchoring surface comprises a porous material (150) that promotes ingrowth of tissue after surgical implantation.

10. A surgical implant according to Claim 1 or Claim 2, wherein the implant (100) has two smooth and lubricious surfaces, and is sized and designed to replace meniscal or labrum tissue.

11. A surgical implant according to Claim 1 or Claim 2, wherein the synthetic polymer is selected from the group consisting of polyacrylonitrile and polyurethane compounds and derivatives.

12. A method for manufacturing a non-resorbable surgical implant (100) for replacing cartilage in a mammalian joint, **characterized in that** said method comprises the following steps:
a. creating a hydrogel material having at least one smooth surface (112) for articulation after surgical implantation, and
b. subjecting at least one smooth surface of the hydrogel material to a chemical treatment that imparts a negative electrical charge to the smooth surface (112), in a manner that creates a charge density that promotes lubricious interactions between the smooth surface (112) and mammalian synovial fluid.

13. The method of Claim 12, wherein the chemical treatment creates a negative electrical charge density on the smooth surface within a range of about -50 to about -250 millimolar, when measured by sodium equilibration.

14. The method of Claim 12, wherein a fibrous reinforcing matrix extends through at least a portion of the hydrogel material.

15. The method of Claim 12, wherein the hydrogel material is made from a synthetic polymer selected from the group consisting of polyacrylonitrile and polyurethane compounds and derivatives.

## Patentansprüche

1. Chirurgisches Implantat (100) zum Ersatz von Knorpel in einem Säugetier-Gelenk, aufweisend ein Hydrogelmaterial, das ein synthetisches Polymer enthält,
**dadurch gekennzeichnet, dass** das chirurgische Implantat (100) mindestens eine glatte und schmierfähige Oberfläche (112) mit einer negativen elektrischen Ladungsdichte hat, die schmierende Wechselwirkungen mit Säugetier-Synovialflüssigkeit fördert.

2. Chirurgisches Implantat (100) gemäß Anspruch 1, wobei eine flexible, fibröse Verstärkungsmatrix (122) in mindestens einem Teil des Hydrogelmaterials eingebettet ist.

3. Chirurgisches Implantat (100) gemäß Anspruch 1 oder Anspruch 2, wobei die glatte und schmierfähige Oberfläche (112) eine negative elektrische Ladungsdichte gemessen durch Äquilibrierung mit Natrium innerhalb eines Bereichs von ungefähr -50 bis ungefähr -250 mmol hat.

4. Chirurgisches Implantat (100) gemäß Anspruch 1 oder Anspruch 2, wobei die glatte und schmierfähige Oberfläche (112) Schwefelatome enthält.

5. Chirurgisches Implantat (100) gemäß Anspruch 1 oder Anspruch 2, wobei die glatte und schmierfähige Oberfläche (112) Fluoratome enthält.

6. Chirurgisches Implantat (100) gemäß Anspruch 1 oder Anspruch 2, wobei die glatte und schmierfähige Oberfläche (112) mit einem chemischen Reagens behandelt wurde, das Vernetzungsverbindungen zwischen Polymermolekülen herstellt.

7. Chirurgisches Implantat (100) gemäß Anspruch 1 oder Anspruch 2, wobei das Implantat eine Verankerungsoberfläche hat, die mit Mitteln zum Verankern des Implantats auf einer präparierten Knochenoberfläche ausgestattet ist.

8. Chirurgisches Implantat (100) gemäß Anspruch 7, wobei die Mittel zum Verankern des Implantats auf einer präparierten Knochenoberfläche Stifte (170) beinhaltet, die dazu ausgelegt sind, in entsprechende Aufnahmen (180) zu passen, die vor dem Einsetzen des chirurgischen Implantats (100) in ein Gelenk in eine präparierte Knochenoberfläche eingesetzt werden können.

9. Chirurgisches Implantat (100) gemäß Anspruch 7, wobei mindestens ein Teil der Verankerungsoberfläche ein poröses Material (150) aufweist, das nach der chirurgischen Implantierung ein Einwachsen von Gewebe fördert.

10. Chirurgisches Implantat gemäß Anspruch 1 oder Anspruch 2, wobei das Implantat (100) zwei glatte und schmierfähige Oberflächen hat und so abgemessen und ausgelegt ist, dass es Meniskus- oder Labrumgewebe ersetzt.

11. Chirurgisches Implantat gemäß Anspruch 1 oder Anspruch 2, wobei das synthetische Polymer aus der Gruppe ausgewählt ist, die aus Polyacrylnitril- und Polyurethanverbindungen und -derivaten besteht.

12. Verfahren zur Herstellung eines nicht-resorbierbaren chirurgischen Implantats (100) zum Ersetzen von Knorpel in einem Säugetier-Gelenk, **dadurch gekennzeichnet, dass** das Verfahren die folgenden Schritte aufweist:
a. Erzeugen eines Hydrogelmaterials, das mindestens eine glatte Oberfläche (112) zur Herstellung einer Gelenkigkeit nach der chirurgischen Implantierung aufweist, und
b. Unterziehen mindestens einer glatten Oberfläche des Hydrogelmaterials einer chemischen Behandlung, die der glatten Oberfläche (112) eine negative elektrische Ladung verleiht, und zwar in einer Weise, die eine Ladungsdichte erzeugt, die schmierende Wechselwirkungen zwischen der glatten Oberfläche (112) und Säugetier-Synovialflüssigkeit fördert.

13. Verfahren gemäß Anspruch 12, wobei die chemische Behandlung auf einer glatten Oberfläche eine negative elektrische Ladungsdichte gemessen durch Äquilibrierung mit Natrium innerhalb eines Bereichs von ungefähr -50 bis ungefähr -250 mmol erzeugt.

14. Verfahren gemäß Anspruch 12, wobei sich eine fibröse Verstärkungsmatrix durch mindestens einen Teil des Hydrogelmaterials erstreckt.

15. Verfahren gemäß Anspruch 12, wobei das Hydrogelmaterial aus einem synthetischen Polymer hergestellt ist, das aus der Gruppe ausgewählt ist, die aus Polyacrylnitril- und Polyurethanverbindungen und -derivaten besteht.

## Revendications

1. Implant chirurgical (100) destiné à remplacer un cartilage dans une articulation de mammifère, ledit implant comprenant un matériau d'hydrogel contenant un polymère synthétique,
**caractérisé en ce que** ledit implant chirurgical (100) a au moins une surface lisse et lubrifiée (112) avec une densité de charge électrique négative qui promeut les interactions lubrifiées avec le fluide synovial de mammifère.

2. Implant chirurgical (100) selon la revendication 1, dans lequel une matrice de renforcement fibreuse flexible (122) est intégrée à l'intérieur d'au moins une partie du matériau d'hydrogel.

3. Implant chirurgical (100) selon la revendication 1 ou la revendication 2, dans lequel la surface lisse et lubrifiée (112) a une densité de charge électrique négative dans une plage d'environ -50 à environ -250 millimolaires, mesurée par équilibration en sodium.

4. Implant chirurgical (100) selon la revendication 1 ou la revendication 2, dans lequel la surface lisse et lubrifiée (112) contient des atomes de soufre.

5. Implant chirurgical (100) selon la revendication 1 ou la revendication 2, dans lequel la surface lisse et lubrifiée (112) contient des atomes de fluor.

6. Implant chirurgical (100) selon la revendication 1 ou la revendication 2, dans lequel la surface lisse et lubrifiée (112) a été traitée avec un réactif chimique qui crée des liaisons de réticulation entre des molécules polymères.

7. Implant chirurgical (100) selon la revendication 1 ou la revendication 2, dans lequel l'implant a une surface d'ancrage dotée de moyens destiné à ancrer l'implant à une surface osseuse préparée.

8. Implant chirurgical (100) selon la revendication 7, dans lequel les moyens d'ancrage de l'implant à une surface osseuse préparée comprennent des broches (170) qui sont conçues pour s'ajuster dans des réceptacles de réception (180) qui peuvent être insérés dans une surface osseuse préparée avant l'insertion de l'implant chirurgical (100) dans une articulation.

9. Implant chirurgical (100) selon la revendication 7, dans lequel au moins une partie de la surface d'ancrage comprend un matériau poreux (150) qui promeut la croissance de tissu après une implantation chirurgicale.

10. Implant chirurgical selon la revendication 1 ou la revendication 2, dans lequel l'implant (100) a deux surfaces lisses et lubrifiées, et est dimensionné et conçu pour remplacer le tissu méniscal ou le bourrelet péri-articulaire.

11. Implant chirurgical selon la revendication 1 ou la revendication 2, dans lequel le polymère synthétique est choisi dans le groupe constitué de composés et de dérivés de polyacrylonitrile et de polyuréthane.

12. Procédé de fabrication d'un implant chirurgical non résorbable (100) destiné à remplacer un cartilage dans une articulation de mammifère, **caractérisé en ce que** ledit procédé comprend les étapes suivantes, consistant à :
a. créer un matériau d'hydrogel ayant au moins une surface lisse (112) pour l'articulation après l'implantation chirurgicale, et
b. soumettre au moins une surface lisse du matériau d'hydrogel à un traitement chimique qui impartit une charge électrique négative à la surface lisse (112), d'une manière qui crée une densité de charge qui promeut des interactions lubrifiées entre la surface lisse (112) et le fluide synovial de mammifère.

13. Procédé selon la revendication 12, dans lequel le traitement chimique crée une densité de charge électrique négative sur la surface lisse dans une plage d'environ -50 à environ -250 millimolaires, mesurée par équilibration en sodium.

14. Procédé selon la revendication 12, dans lequel une matrice de renforcement fibreuse s'étend à travers au moins une partie du matériau d'hydrogel.

15. Procédé selon la revendication 12, dans lequel le matériau d'hydrogel est formé à partir d'un polymère synthétique choisi dans le groupe constitué de composés et de dérivés de polyacrylonitrile et de polyuréthane.
